# EUROPEAN PATENT SPECIFICATION

(11) **EP 4 400 118 B1**
(45) Date of publication and mention of the grant of the patent: **29.07.2026**
(21) Application number: 22867390.1
(22) Date of filing: 07.09.2022
(51) Int. Cl.: A61K 45/00, A61K 31/56, A61K 31/727, A61K 38/07, A61K 38/08, A61K 38/12, A61P 13/10, A61P 29/00, A61P 43/00

(54) **PHARMACEUTICAL COMPOSITION FOR PREVENTING OR TREATING INTERSTITIAL CYSTITIS/BLADDER PAIN SYNDROME**
PHARMAZEUTISCHE ZUSAMMENSETZUNG ZUR PRÄVENTION ODER BEHANDLUNG DES INTERSTITIELLEN ZYSTITIS/BLASENSCHMERZSYNDROMS
COMPOSITION PHARMACEUTIQUE DESTINÉE À LA PRÉVENTION OU AU TRAITEMENT DU SYNDROME DE LA CYSTITE INTERSTITIELLE/DOULEUR DE LA VESSIE

(30) Priority: 07.09.2021 JP 2021145762
(43) Date of publication of application: 17.07.2024
(73) Proprietor: Public University Corporation Nagoya City University, Mizuho-ku Nagoya-shi Aichi 467-8601 (JP); National University Corporation Asahikawa Medical University, Asahikawa, Hokkaido 078-8510 (JP)
(72) Inventor: HOTTA Yuji, Nagoya-shi, Aichi 467-8603 (JP); KIMURA Kazunori, Nagoya-shi, Aichi 467-8603 (JP); KAWATA Ryoya, Nagoya-shi, Aichi 467-8603 (JP); MATSUMOTO Seiji, Asahikawa-shi, Hokkaido 078-8510 (JP)
(74) Representative: Wasner, Marita
(86) International application number: PCT/JP2022/033623
(87) International publication number: WO 2023/038068

(56) References cited:
- JP-A- 2020 502 125
- US-A1- 2013 058 873
- US-A1- 2018 344 803
- US-A1- 2020 323 772
- US-B2- 10 836 720
- PRICE N., CARVER J, JACKSON S, MARDON H: "INVESTIGATION OF THE FUNCTION OF INTEGRIN-MEDIATED BACTERIAL ADHERENCE PROTEINS AND THEIR RECEPTORS IN WOMEN WITH RECURRENT BACTERIAL CYSTITIS AND INTERSTITIAL CYSTITIS", 39TH ANNUAL MEETING OF THE INTERNATIONAL CONTINENCE SOCIETY, 1 January 2009 (2009-01-01), XP093045733
- CARLOS MAS‐MORUNO; ROBERTA FRAIOLI; FLORIAN RECHENMACHER; STEFANIE NEUBAUER; TOBIAS G. KAPP; HORST KESSLER: "αvβ3‐ or α5β1‐Integrin‐Selective Peptidomimetics for Surface Coating", ANGEWANDTE CHEMIE INTERNATIONAL EDITION, VERLAG CHEMIE, HOBOKEN, USA, vol. 55, no. 25, 3 June 2016 (2016-06-03), Hoboken, USA, pages 7048 - 7067, XP072068485, ISSN: 1433-7851, DOI: 10.1002/anie.201509782

## Description

### Field

The present invention relates to a pharmaceutical composition for the treatment of interstitial cystitis/bladder pain syndrome, containing a certain RGD integrin inhibitor.

The invention is defined in the claims.

### Background

Interstitial cystitis/bladder pain syndrome (IC/BPS) is defined as "a condition of chronic pelvic pain, pressure, or discomfort related to the bladder, accompanied by lower urinary tract symptoms such as increased desire to urinate and frequent urination, without potentially confusing disease" (Non-Patent Literature 1). IC/BPS with erosive lesions (Hunner's lesions) is called Hunner's interstitial cystitis. Hunner's interstitial cystitis has clear abnormal findings both endoscopically and pathologically, is considered more severe symptomatically, and is designated as Intractable Disease No. 226 by the Ministry of Health, Labour and Welfare in Japan.

There is still no established method for the treatment of IC/BPS, and it currently remains limited to symptomatic treatment. Surgical interventions include endoscopic bladder hydrodistention, and when Hunner's lesion is identified in the bladder, electrical or laser cauterization is also performed concurrently. Pharmacological treatments include oral administration of central sensitizers such as amitriptyline, or immunosuppressive agents such as cyclosporine A, as well as intravesical instillation of drugs such as heparin, DMSO, or steroids, and injection of botulinum toxin into the bladder wall.

Currently, bladder hydrodistention or cautery of Hunner's lesions provides symptom remission in approximately half of the cases, however, long-term remission is confined to a limited number of cases. Furthermore, pharmacological treatments have not achieved a cure for IC/BPS. Thus, IC/BPS is a refractory quality-of-life disease requiring long-term medical management due to its recurrent nature of relapses and remissions, and there is a significant demand for the development of effective therapeutics.

### Citation List

### Non-Patent Literature

Non-Patent Literature 1: Clinical Guideline for Interstitial Cystitis/Bladder Pain Syndrome, edited by the Society of Interstitial Cystitis of Japan/the Japanese Urological Association, published on April 25, 2019.
Price N et al. relates to function of integrin-mediated bacterial adherence proteins and their receptors.

### Summary

### Technical Problem

The objectives of the present invention include providing a novel therapeutic approach for IC/BPS.

### Solution to the Problem

The inventors have found that a certain RGD integrin inhibitor is useful for treating IC/BPS, in particular for ameliorating frequent urination and pain.

### Advantageous Effect

According to the present invention, the treatment of IC/BPS based on a novel mechanism of action can be achieved.

### Brief Description of Drawings

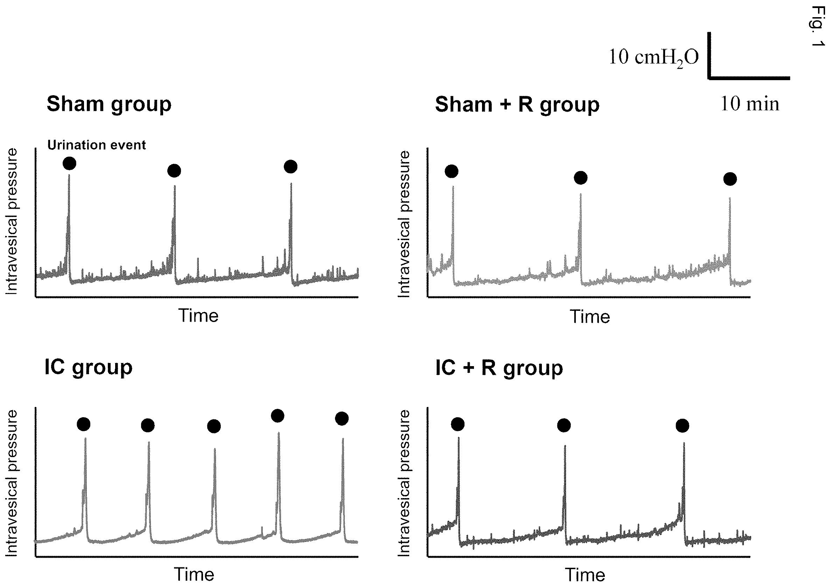

[FIG. 1] FIG. 1 includes graphs illustrating the cystometrograms in IC/BPS model and control rats, each administered intraperitoneally either saline or a peptide (RGDS peptide) consisting of the amino acid sequence as shown in SEQ ID NO: 1 (n=7-8 for each group). Sham denotes control rats that were administered saline, Sham+R denotes control rats that were administered RGDS peptide, IC denotes IC/BPS model rats that were administered saline, and IC+R denotes IC/BPS model rats that were administered RGDS peptide. The vertical axes represent intravesical pressure and the horizontal axes represent elapsed time.
[FIG. 2] FIG. 2 includes graphs illustrating the urination intervals, urine volume per urination event, maximum intravesical pressure, threshold pressure for urination, and basal intravesical pressure in IC/BPS model and control rats, each administered intraperitoneally either saline or RGDS peptide.
[FIG. 3] FIG. 3 includes a graph illustrating the urination intervals in IC/BPS model rats administered intraperitoneally saline, RGDS peptide, or cilengitide (n=5-8).
[FIG. 4] FIG. 4 includes a graph illustrating the pain thresholds in IC/BPS model and control rats, each administered intraperitoneally either saline or RGDS peptide (n=2-4).

### Description of Embodiments

The following description may be based on representative embodiments or specific examples, however, the present invention is not to be construed as limited to such embodiments or specific examples. In the present specification, a numerical range expressed with "to" or "-" means a range including numerical values at both ends thereof as an upper limit value and a lower limit value, unless otherwise noted.

The present disclosure provides a pharmaceutical composition for the prevention or treatment of IC/BPS, containing an inhibitor against at least one type of integrin selected from the group consisting of integrin αvβ3, integrin αvβ5, and integrin α5β1.

Integrins are heterodimeric receptors consisting of two subunits, an α-chain and a β-chain. At least 18 different α-subunits and 8 different β-subunits have been identified in mammals and are known to form more than 24 heterodimers. Eight subtypes of integrins, namely integrin avβ1 (hereafter simply referred to as αvβ1, the same applies to other integrins), αvβ3, αvβ5, αvβ6, αvβ8, αllbβ3, α8β1, and α5β1, recognize the amino acid sequence Arg-Gly-Asp (RGD motif) and bind peptides having this sequence. These integrins are referred to as RGD integrins or RGD-binding integrins.

RGD integrins have various physiological functions such as in tissue repair, angiogenesis, inflammation, thrombogenesis, and the promotion of tumor growth and metastasis. Regulating RGD integrin functions through inhibition of binding to peptide ligands with RGD motifs is expected to be effective in the treatment of diseases such as cancer and autoimmune diseases. In light of these expectations, numerous compounds that mimic the structure of RGD, also called RGD mimics or RGD mimetics, specific antibodies, and various other forms of inhibitors against RGD integrin have been found and reported. The present disclosure relates to a new use for an inhibitor against at least one integrin selected from the group consisting of αvβ3, αvβ5, and α5β1.

The inhibitor against at least one integrin selected from the group consisting of αvβ3, αvβ5 and α5β1 (hereinafter referred to as RGD integrin inhibitor) refers to a substance that has the activity to inhibit the binding of at least one integrin selected from the group consisting of αvβ3, αvβ5, and α5β1 to its ligand, which is a peptide having an RGD motif, such as vitronectin or fibronectin. The RGD integrin inhibitor has the ability to inhibit the binding of at least one integrin selected from the group consisting of αvβ3, αvβ5, and α5β1, to a peptide having an RGD motif with an IC₅₀ of 1000 nM or less, preferably 500 nM or less, more preferably 200 nM or less, preferably 500 nM or less, more preferably 200 nM or less, and furthermore preferably 100 nM or less.

The RGD integrin inhibitor can be a peptide, protein, glycoprotein, nucleic acid, low molecular weight organic compound, or other compounds. Substances that inhibit the binding of integrin to its ligand may also be referred to as integrin antagonists. These integrin antagonists are included in the RGD integrin inhibitor as long as they have the activity to inhibit the binding of at least one integrin selected from the group consisting of αvβ3, αvβ5, and α5β1 to its ligand.

Examples of an RGD peptide and RGD mimic that can be used as the RGD integrin inhibitor can include, but are not limited to, cilengitide (CAS No. 188968-51-6), GLPG0187 (CAS No. 1320346-97-1), JSM-6427 (CAS No.), GSK3008348 (CAS No. 1629249-33-7), MK-0429 (CAS No. 227963-15-7), ATN-161 (CAS No. 262438-43-7), CWHM 12 (CAS No. 1564286-55-0), SB-273005 (CAS No. 205678-31-5), SB-267268 (CAS No. 205678-26-8), SC-68448 (CAS No. 188804-07-1), Compound 11 (CAS No. 287961-15-3), Example 1 (CAS No. 1893398-07-6), echistatin, RGD, RGDS (peptide consisting of the amino acid sequence as shown in SEQ ID NO: 1), GRGD (peptide consisting of the amino acid sequence as shown in SEQ ID NO: 2), GRGDS (peptide consisting of the amino acid sequence as shown in SEQ ID NO: 3), GRGDSP (peptide consisting of the amino acid sequence as shown in SEQ ID NO: 4), GRGDSPK (peptide consisting of the amino acid sequence as shown in SEQ ID NO: 5), GRGDNP (peptide consisting of the amino acid sequence as shown in SEQ ID NO: 6), GRGDTP (peptide consisting of the amino acid sequence as shown in SEQ ID NO: 7), c(RGDfV), c(RGDfK), c(RGDyK), c(RGDfC), sn243, RGD-4C, RGD10, NC100717, c(phgisoDGRk), 44b, Mol 11, F-Galacto-c(RGDfK), (Ga)NOPO-c(RGDfK), c(RGDfK)-Peg-MPA, Flucilatide, c(phgisoDRGk)-Peg-MPA.

An antibody or derivative thereof that specifically binds to at least one integrin selected from the group consisting of αvβ3, αvβ5, and α5β1, thereby inhibiting its binding to its ligand can also be used as the RGD integrin inhibitor. The specific antibody against at least one integrin selected from the group consisting of αvβ3, αvβ5, and α5β1 (termed the target integrin) can also be represented as an antibody that preferentially binds to the target integrin over non-target proteins, or an antibody with high binding affinity to the target integrin. The specific antibody can bind to the target integrin with affinity at least 5 times stronger, preferably at least 10 times stronger, more preferably at least 100 times stronger, and most preferably at least 1000 times stronger than affinity to the non-target proteins. The specific antibody can also be an antibody that binds to the target integrin with a dissociation constant of 10⁻⁷ M, preferably 10⁻⁸ M, more preferably 10⁻⁹ M.

The specific antibody may originate from any species including, for example, mice, rats, sharks, rabbits, pigs, hamsters, camels, llamas, goats, or humans. The specific antibody can be of any immunoglobulin class (for example, IgG, IgE, IgM, IgD, or IgA) and subclass, and IgG is preferred.

The specific antibody may be a polyclonal antibody or a monoclonal antibody, and a monoclonal antibody is preferred. The specific antibody may be a chimeric antibody, a humanized antibody, or a human antibody.

The derivative of the specific antibody may be an antigen-binding fragment of the antibody that is defined as a partial fragment of the antibody having the ability to bind specifically to the antigen of the antibody, including, but not limited to, a fragment of antigen binding (Fab), Fab', F(ab')2, single chain Fv, disulfide stabilized Fv, and a peptide containing the CDRs of the antibody.

The specific antibody and derivative thereof can be produced using methods known to those skilled in the art. For example, they can be produced by preparing at least one type of integrin selected from the group consisting of αvβ3, αvβ5, and α5β1, or a partial peptide having the RGD motif-binding region of the integrin by using genetic recombination techniques based on the amino acid sequence of human integrin or the nucleotide sequence of DNA encoding it, immunizing an appropriate animal with it as an antigen, and then fusing B cells of the animal with myeloma cells to obtain hybridomas. For the hybridoma method, see, for example, Meyaard et al. (1997) Immunity 7:283-290; Wright et al. (2000) Immunity 13:233-242; Kaithamana et al. (1999) J.Immunol. 163:5157-5164. The specific antibody and derivative thereof may be conjugated with other drugs to form antibody-drug conjugates.

Non-limiting examples of the specific antibody against at least one integrin selected from the group consisting of αvβ3, αvβ5, and α5β1 can include Intetumumab (CNTO 95) (CAS No. 725735-28-4), Etaracizumab (MEDI-522, Abergrin) (CAS No. 89255-42-3), PF-04605412 (CAS No. 1677682-18-6), Volociximab (CAS No. 558480-40-3), EMD525797 (abituzumab, DI17E6) (CAS No. 1105038-73-0), Abciximab (ReoPro, c7E3) (CAS No. 143653-53-6), LM609 (MAB1976Z), P5H9 (MAB2528), P5D2 (MAB17781), 272-17E6 (MABT207).

The RGD integrin inhibitor may have the activity to inhibit binding of two or more RGD integrins selected from the group consisting of αvβ3, αvβ5, and α5β1, and may further have the activity to inhibit RGD integrins other than αvβ3, αvβ5 and α5β1.

For example, the RGD integrin inhibitor has the activity to inhibit at least αvβ3, and, for example, the IC₅₀ value of the RGD integrin inhibitor for αvβ3 can be 1000 nM or less, preferably 500 nM or less, more preferably 200 nM or less, furthermore preferably 100 nM or less.

Examples of the RGD integrin inhibitor having the activity to inhibit αvβ3 can include, in the RGD peptides and RGD mimics, cilengitide (CAS No. 188968-51-6), GLPG0187 (CAS No. 1320346-97-1), GSK3008348 (CAS No. 1629249-33-7), MK-0429 (CAS No. 227963-15-7), CWHM 12 (CAS No. 1564286-55-0), SB-273005 (CAS No. 205678-31-5), SB-267268 (CAS No. 205678-26-8), SC-68448 (CAS No. 188804-07-1), Compound 11 (CAS No. 287961-15-3), Example 1 (CAS No. 1893398-07-6), echistatin, RGD, RGDS, GRGD, GRGDS, GRGDSP, GRGDSPK, GRGDNP, GRGDTP, c (RGDfV), c (RGDfK), c (RGDyK), c (RGDfC), sn243, RGD-4C, RGD10, NC100717, Mol 11, F-Galacto-c (RGDfK), (Ga)NOPO-c (RGDfK), c (RGDfK)-Peg-MPA, Flucilatide, and can include, in the specific antibodies against at least one integrin selected from the group consisting of αvβ3, αvβ5, and α5β1, Intetumumab (CNTO 95) (CAS No. 725735-28-4), Etaracizumab (MEDI-522, Abergrin) (CAS No. 89255-42-3), EMD525797 (abituzumab, DI17E6) (CAS No. 1105038-73-0), Abciximab (ReoPro, c7E3) (CAS No. 143653-53-6), LM609 (MAB1976Z), P5H9 (MAB2528), P5D2 (MAB17781), 272-17E6 (MABT207).

The RGD integrin inhibitor is, for example, RGDS peptide or cilengitide.

Other than the RGD integrin inhibitors exemplified above, a substance having the inhibitory activity, as determined by methods known in the art for evaluating inhibitory activity against αvβ3, αvβ5 or α5β1, may also be used as the RGD integrin inhibitor. Such methods involve, for example, comparing the binding of at least one integrin selected from the group consisting of αvβ3, αvβ5, and α5β1 to its ligand in the presence and absence of the test substance, respectively. The measurement of the inhibitory activity can be performed using the amino acid sequences of each subunit of αvβ3, α5β1, and α5β1 and the nucleotide sequences of the genes encoding them, which are registered in public databases such as GenBank, and recombinant cells capable of expressing each integrin, etc. as appropriate, by those skilled in the art within their normal practice capabilities.

The pharmaceutical composition in the present disclosure is a pharmaceutical composition for the prevention or treatment of IC/BPS, containing the RGD integrin inhibitor described above. The pharmaceutical composition in the present disclosure may contain one type of the RGD integrin inhibitors or multiple types of the RGD integrin inhibitors in any proportion. The pharmaceutical composition in the present disclosure may also contain the RGD integrin inhibitor in free form, in the form of an ester, in the form of a salt, or in the form of a mixture thereof, or may contain multiple types of salts or esters, depending on its physical properties and other factors.

The pharmaceutical composition in the present disclosure can be used for the prevention or treatment of IC/BPS. The term "prevention" as used herein encompasses all types of medically acceptable prophylactic interventions aimed at, for example, preventing or inhibiting the onset or development of a disease. The term "treatment" encompasses all types of medically acceptable therapeutic interventions aimed at, for example, curing or temporarily remitting a disease or condition. Thus, the prevention or treatment of IC/BPS encompasses medically acceptable interventions for a variety of purposes, including ameliorating, delaying or stopping the progression, preventing the onset or recurrence of symptoms such as frequent urination, increased desire to urinate, urinary urgency and pain in IC/BPS. The pharmaceutical composition in the present invention can be used for the amelioration of frequent urination or for the amelioration of pain in IC/BPS.

The pharmaceutical composition in the present disclosure contains the RGD integrin inhibitor in an effective amount for the prevention or treatment of IC/BPS. The effective amount for the prevention or treatment of IC/BPS can be determined as appropriate depending on usage, subject age, gender, weight, severity, and other factors.

The pharmaceutical composition in the present disclosure may contain, in addition to the RGD integrin inhibitor, a pharmaceutically acceptable additive. Examples of the pharmaceutically acceptable additive can include a buffer, a stabilizer, a preservative, and an excipient. The pharmaceutically acceptable additive is well known to those skilled in the art and can be selected and used by those skilled in the art within their normal practice capabilities.

The pharmaceutical composition in the present disclosure may contain other medicinal agents for the prevention or treatment of IC/BPS. Examples of the other agents for the prevention or treatment of IC/BPS can include those listed in Non-Patent Literature 1 (Clinical Guideline for Interstitial Cystitis/Bladder Pain Syndrome). In particular, when the pharmaceutical composition in the present disclosure is intended for intravesical instillation, agents suitable for intravesical administration, such as heparin, DMSO, or steroids, are preferred.

There are no specific limitations on the dosage form of the pharmaceutical composition in the present disclosure, and a dosage form suitable for intraperitoneal administration, intravenous administration, or transurethral or transcystostomy intravesical instillation, such as a liquid formulation, is preferred. Such a pharmaceutical composition can be represented as a pharmaceutical composition for the prevention or treatment of IC/BPS to be administered systemically or to be instilled intravesically, containing the RGD integrin inhibitor, i.e., an inhibitor against at least one type of integrin selected from the group consisting of αvβ3, αvβ5, and α5β1.

The liquid formulation contains an effective amount of RGD integrin inhibitor and a pharmaceutically acceptable vehicle. Examples of the pharmaceutically acceptable vehicle can include, for example, an aqueous medium such as water, saline, phosphate buffered saline (PBS). The unit dose of the liquid formulation can be determined depending on the route of administration and the effective amount of RGD integrin inhibitor, for example, 30-50 mL for intravesical instillation, which can be administered to the subject at appropriate intervals, such as once a week, once every other week, or once a month.

The pharmaceutical composition is administered to a subject at a risk of developing or having developed IC/BPS, such as rodents including mice, rats, hamsters, and guinea pigs; primates including humans, chimpanzees, and rhesus monkeys; domestic animals including pigs, cattle, goats, horses, and sheep; and pet animals including dogs and cats. Preferred subjects are humans.

In the explanation above, the parts not falling within the scope of the claims, specifically the parts relating to the prevention of IC/BPS and the parts relating to the RGD integrin inhibitors other than the peptide consisting of the amino acid sequence as shown in SEQ ID NO: 1 and cilengitide are disclosed as references.

The present disclosure further relates to, as a reference, a method for preventing or treating IC/BPS, including administering the pharmaceutical composition containing the RGD integrin inhibitor described above to a subject at risk of developing or having developed IC/BPS. This method is outside the subject matter of the claims. The present disclosure also relates to, as a reference, a method for ameliorating frequent urination or pain in IC/BPS, including administering the pharmaceutical composition containing the RGD integrin inhibitor described above. This method is outside the subject matter of the claims.

Moreover, the present disclosure relates to, as a reference, use of the RGD integrin inhibitor described above for the manufacture of the pharmaceutical composition for the prevention or treatment of IC/BPS; use of the RGD integrin inhibitor described above for the manufacture of the pharmaceutical compositions for the amelioration of frequent urination or pain in IC/BPS; use of the RGD integrin inhibitor described above for the prevention or treatment of IC/BPS; and use of the RGD integrin inhibitor described above for the amelioration of frequent urination or pain in IC/BPS. These uses are outside the subject matter of the claims.

Furthermore, the present disclosure relates to, in another aspect, as a reference, a method for evaluating the effect of a substance being tested on interstitial cystitis/bladder pain syndrome, the method including using, as an indicator, the activity of the substance to inhibit at least one type of integrin selected from the group consisting of αvβ3, αvβ5, and α5β1 (hereinafter referred to as "the evaluation method"). This method is outside the subject matter of the claims. A substance that is evaluated as effective by the evaluation method is expected to be a substance that can be used for prevention or treatment of IC/BPS through its activity to inhibit at least one integrin selected from the group consisting of αvβ3, αvβ5, and α5β1.

One example of the evaluation method is a method known in the art for evaluating the inhibitory activity against αvβ3, αvβ5 or α5β1, for example, a method including comparing the binding of at least one integrin selected from the group consisting of αvβ3, αvβ5, and α5β1 to its ligand in the presence and absence, respectively, of a substance to be tested, thereby evaluating the substance that is determined to have the inhibitory activity as effective for the prevention or treatment of IC/BPS. The measurement of the inhibitory activity can be performed using the amino acid sequences of each subunit of αvβ3, α5β1, and α5β1 and the nucleotide sequences of the genes encoding them, which are registered in public databases such as GenBank, and recombinant cells capable of expressing each integrin, etc. as appropriate, by those skilled in the art within their normal practice capabilities.

While the present invention will be described in more detail by the following examples, it should not be considered as being limited to these examples.

### [Examples]

### Example 1

Ten-week-old female F344/NSIC344 rats (weighing approximately 140-170 g) had a polyethylene catheter inserted and retained in the urethra. Through the catheter, 250 µL of saline (Sham group) or 250 µL of 0.1 M HCl (IC group) was instilled into the bladder and held for 1 minute, and then the bladder was washed twice with saline. The Sham and IC groups were each divided into two groups (n=7-8/group), and 200 µL of saline or 200 µL of RGDS peptide solution (500 mg/L saline solution, MedChemExpress) was administered intraperitoneally. On day 7 after administration, a polyethylene catheter was inserted and retained in the apex of the bladder, and a pressure transducer (MLT0699, ADInstruments Ltd, Dunedin, New Zealand), PowerLab 4/26 (ADInstruments Ltd, Dunedin New Zealand) were connected. A syringe pump (YSP-101, YMC Co., Ltd., Kyoto, Japan) was connected to the pressure transducer to measure intravesical pressure and urination volume while continuously instilling 40 µL/min saline solution into the bladder. The pressure at the urination event peak on the cystometrogram was defined as the maximum intravesical pressure, the peak interval as the urination interval, the pressure immediately after the peak as the basal intravesical pressure, the pressure immediately before the peak as the threshold pressure for urination, and the urination volume at the peak as urine volume per urination event.

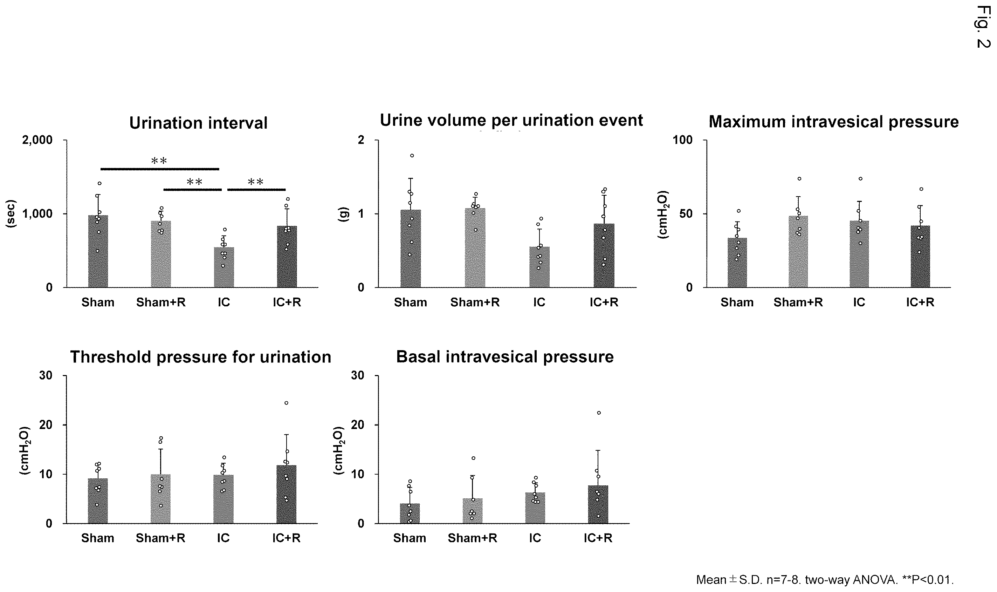

In the IC group that received instillation of RGDS peptide (IC+R group), the urination interval was significantly prolonged compared to the IC group that received instillation of saline (Figures 1 and 2), confirming that the frequent urination was ameliorated. The average urine volume per urination event in the IC+R group was similar to that in the Sham group, and the maximum intravesical pressure, basal intravesical pressure, and threshold pressure for urination did not differ among all groups, indicating that the RGDS peptide had no effect on these parameters.

### Example 2

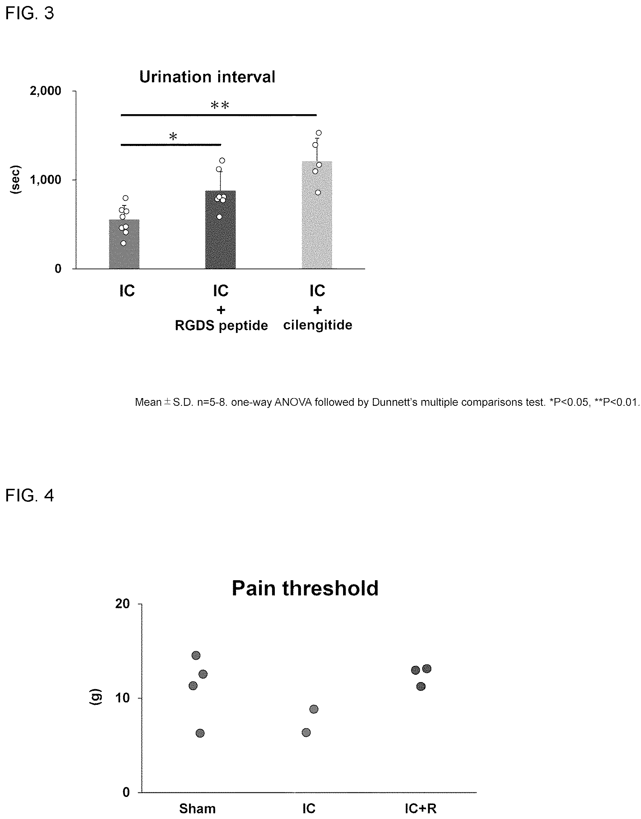

A similar study to Example 1 was conducted, except that the RGDS peptide used in Example 1 was replaced with 400 µL of cilengitide solution (6.75 g/L saline solution, Tokyo Kasei). In the cilengitide-administered group, the urination interval was prolonged compared to the group that received instillation of saline (Figure 3), confirming that frequent urination was ameliorated.

Sham, IC, and IC+R groups were prepared as in Example 1. On day 5 after administration, the rats were placed in a metabolic cage with a wire mesh bottom and allowed to acclimate for 10 minutes, and the lower abdomen was stimulated with increasing pressure using an electronic von Frey filament (BIO-EVF, Bioseb, Vitrolles, France). The pressure at which an escape response was observed was measured as the pain threshold. This measurement was repeated three times with a 5-minute interval, and the average was calculated. The pain threshold increased in the IC+R group compared to the IC group (Figure 4), confirming an ameliorating trend of pain.

## Claims

1. A pharmaceutical composition for use in the treatment of interstitial cystitis/bladder pain syndrome, containing a peptide consisting of the amino acid sequence as shown in SEQ ID NO: 1, or cilengitide.

2. The pharmaceutical composition for use according to claim 1, for ameliorating frequent urination or pain in interstitial cystitis/bladder pain syndrome.

3. The pharmaceutical composition for use according to claim 1 or 2, being to be instilled intravesically.

4. The pharmaceutical composition for use according to any one of claims 1 to 3, further containing another agent for the treatment of interstitial cystitis/bladder pain syndrome.

5. The pharmaceutical composition for use according to claim 4, wherein the another agent is heparin, DMSO, or a steroid.

## Patentansprüche

1. Pharmazeutische Zusammensetzung zur Verwendung bei der Behandlung von interstitieller Zystitis/Blasenschmerzsyndrom, enthaltend ein Peptid, das aus der in SEQ ID NO: 1 gezeigten Aminosäuresequenz besteht, oder Cilengitid.

2. Pharmazeutische Zusammensetzung zur Verwendung gemäß Anspruch 1, zur Linderung von häufigem Harndrang oder Schmerzen bei interstitieller Zystitis/Blasenschmerzsyndrom.

3. Pharmazeutische Zusammensetzung zur Verwendung gemäß Anspruch 1 oder 2, die intravesikal instilliert wird.

4. Pharmazeutische Zusammensetzung zur Verwendung gemäß einem der Ansprüche 1 bis 3, die ferner einen weiteren Wirkstoff zur Behandlung von interstitieller Zystitis/Blasenschmerzsyndrom enthält.

5. Pharmazeutische Zusammensetzung zur Verwendung gemäß Anspruch 4, wobei der weitere Wirkstoff Heparin, DMSO oder ein Steroid ist.

## Revendications

1. Composition pharmaceutique destinée à être utilisée dans le traitement de la cystite interstitielle/du syndrome de douleur de la vessie, contenant un peptide constitué de la séquence d'acides aminés représentée dans SEQ ID NO : 1, ou du cilengitide.

2. Composition pharmaceutique destinée à être utilisée selon la revendication 1, pour améliorer la miction fréquente ou la douleur associée à la cystite interstitielle/au syndrome de douleur de la vessie.

3. Composition pharmaceutique destinée à être utilisée selon la revendication 1 ou 2, devant être instillée par voie intravésicale.

4. Composition pharmaceutique destinée à être utilisée selon l'une quelconque des revendications 1 à 3, contenant en outre un autre agent pour le traitement de la cystite interstitielle/du syndrome de douleur de la vessie.

5. Composition pharmaceutique destinée à être utilisée selon la revendication 4, dans laquelle l'autre agent est l'héparine, le DMSO ou un stéroïde.
